# EUROPEAN PATENT APPLICATION

(11) **EP 0 577 053 A1**
(43) Date of publication of application: **05.01.1994**
(21) Application number: 93110303.0
(22) Date of filing: 28.06.1993
(51) Int. Cl.: A61B 5/083, A61B 5/097

(54) **Expired gas sampling method and expired gas collecting tube**

(30) Priority: 30.06.1992 JP 197698/92
(71) Applicant: Ueda, Hideo, Minoh-shi, Osaka (JP); KABUSHIKI KAISHA KYOTO DAIICHI KAGAKU, Kyoto-shi Kyoto 601 (JP)
(72) Inventor: Ueda, Hideo, Minoh-shi, Osaka (JP); Hiromoto, Mitsuo, Uji-shi, Kyoto (JP)
(74) Representative: Dipl.-Ing. H. Hauck, Dipl.-Ing. E. Graalfs, Dipl.-Ing. W. Wehnert, Dr.-Ing. W. Döring, Dr.-Ing. N. Siemons

(57) **Abstract**

A gas collecting tube (1) is used for sampling expired gas for preventing adsorption or contamination of aimed gas components contained therein and for enabling measurement with excellent reproducibility.

The expired gas collecting tube (1) comprises a heating type flexible tube which includes an elastic tube superior in heat-resistance and provided circumferentially or inside with a heating element (3) on which a heat insulating material (4) covers, or a heating type hard tube made of metal or thermosetting resin and provided circumferentially or inside with a heating element on which a heat insulating material covers, and the heating type flexible tube or the heating type hard tube being provided at utmost end with an adapter (8) for mounting an expired gas collecting mask or mouthpiece (9).

## Description

### BACKGROUND OF THE INVENTION

The invention relates to an expired gas sampling method in association with a clinical examination device using expired gas as samples and an expired gas collecting tube for use in the sampling method.

A clinical biochemical examination device using expired gas (expired air) as samples has not been put to practical use to the best of our knowledge. This is because analytical technology of blood and urine has been highly developed in the field of the clinical examination. However, collecting blood causes subjects or patients a physical pain with loss of their important blood, and repeated collection of blood (self-monitoring of diabetes or the like) and a continuous measurement of blood (blood sugar, etc) impose a heavy burden on the subjects or patients. Also, urine, the typical samples for bloodless and non-invasive clinical examinations, provides less information than blood does. The examinations with urine cause the subjects or patients a mental pain as from a feeling of shame and do not enable a continuous measurement, and urine is hard to be collected at all times. Further, in the clinical examinations with blood or urine, easily vaporized components such as ammonia, acetone or the like cannot be accurately measured.

Under the circumstance, other clinical analyzing techniques in bloodless and noninvasive type than the urine analyzing technique, for example, those using as samples such body fluid other than blood as exudate from skin (lymph), sweat, saliva or the like, or percutaneously measuring blood gas with a blood gas sensor, have been hitherto studied. But, these techniques have not been broadly put into practical use due to the facts that merely less amount of samples are obtainable and measurable items are limited.

Hence, the inventors started developing a novel clinical examination technology with giving special notice to expired gas which is available in a bloodless and noninvasive manner. Expired gas is intermittently breathed out by human (or animals) during their lives are maintained, and it is readily collectable, without causing subjects a physical or mental pain. Also, since trace amounts of volatile component of mixed venous blood flowing through alveolar blood capillary is moved into expired air by gas exchange, it is inferred that expired air and blood have correlation with respect to the volatile component. Furthermore, examinations with expired gas enables differential measurement of the volatile component which measurement the blood analysis could not cover. Thus, expired gas (expired air) is an ideal sample for the clinical biochemical examination.

Since gases of the expired air to be detected (those having importance in clinical examinations) are quite low in concentration (ppb or ppm at the most), they could be measurable only by a combination of a concentrating device of the trace amounts of gas component and a large-scaled high-sensitivity gas detecting device and skilled operators. There are few clinical reports in this kind of basic analysis of expired gas by use of the above device. Such clinical reports the present inventor knows are (1) Dubowski, K,M, Breath Analysis as a Technique in Clinical Chemistry, Clin .Chem., 20.966-972, 1974, (2) Manolis,A., The Diagnostic Potential of Breath Analysis, Clin.Chem., 29. 5-15, 1983, and (3) Phillips, M., Breath Tests In Medician, Scientific American July.1992. However, the techniques reported in those reports cannot be made use or for such as a bedside examination, a pre-hospital examination in an ambulance, screening upon medical examination or the like. Furthermore, expired gas when collected in vessels occupies much spaces for preservation and transportation and some gas components subject to examination are unstable, so that they are not well subjected to the analyzing course as of blood, i.e., to be first transported to the analysis center and analyzed by large-scaled analytical apparatuses. Hence, conditions for a practical clinical biochemical examination device are to be small-sized and portable, having high sensitivity and high accuracy and readily operable.

The inventors zealously studied under the foregoing circumstances and succeeded in davelopment of a portable type examination device which can measure aimed gas components of expired gas with high sensitivity and high accuracy according to some measuring principles. Expired gas when breathed out disperses instantly into atmosphere. To feed the expired gas samples to the measuring part in the examination device with the expired gas being in the state not mixing with ambient air, we adopted an expired air collecting tube provided with an expired gas collecting mask or mouthpiece. We continuously tested the examination device found problems of scattering results of measuring data and a poor reproducibility or the like.

To investigate causes of the problems, we prepared an aqueous solution of a sample gas such as ammonia and placed the solution in a vessel. The expired gas collecting tube was used to suck in the gas gathered in the head space of the vessel and continuously measure the gas in batching manner. Measured values gradually lowered and became stable at a value lowered than an initial value after a plural times of measurement. Then, we removed the collecting tube from the vessel and made the measurement and found that the gas was detected in each measurement although measured values were rather lower than the initial value and gradually lowered. Thereafter, when the collecting tube was removed from the measuring device, measured value became zero instantly. In principle, the measured value should become zero at the time when the collecting tube is removed form the vessel with the exception of time lag that the gas remaining in the tube vanishes (and guessing that no measurable gases exist in atmosphere).That phenomenon is inferred to be from that moisture vapor adhered on an inner wall of the collecting tube to form a layer of moisture on the inner wall and the sample gas dissolved in the moisture layer. From the above, it has been revealed that Expired gas contains a considerable amount of moisture vapor, and gas components of expired air to be detected are in quite trace amounts and fully hydrophilic because they are in vivo substances, so that measurement of expired gas is largely influenced when a quite little amount of moisture adheres to the transmitting system of expired gas. The conventional expired gas collecting tube does not avoid influences by contamination as above and not guarantee reproducibility.

### SUMMARY OF THE INVENTION

An object of the invention is to provide an expired gas sampling method in association with a clinical examination device using expired gas as samples and an expired gas collecting tube for use in the sampling method. Also, an object of the invention is to provide a heating type of expired gas sampling method and expired gas collecting tube for preventing adhesion of moisture contained in expired gas on inner wall or the tube. Further, an object of the invention is to provide an expired gas collecting tube provided with a mounting part for mounting an expired gas collecting mask or mouthpiece.

### BRIEF DESCRIPTION OF THE DRAWINGS

Fig. 1 shows an example of an expired gas collecting tube according to the present invention, Fig. 1 (a) being a partially sectional side view and Fig. 1(b) a sectional view taken in the line X-X in Fig.1(a).

Fig. 2 is a schematic diagram showing the expired gas collecting tube connected to a main body of an examination device.

Fig. 3 is a schematic diagram showing a modified example of the expired gas collecting tube connected to a main body of an examination device.

Fig. 4 is a schematic diagram showing a further modified example of the expired gas collecting tube connected to a main body of an examination device.

Fig. 5 is a partially sectional side view showing a modified example of the expired gag collecting tube.

Fig. 6 is a partially sectional side view showing a further modified example of the expired gas collecting tube.

### DETAILED DESCRIPTION OF THE INVENTION

The inventors have zealously studied under the foregoing circumstances an expired gas sampling device free of influence by moisture contained in expired gas and achieved the present invention. The invention relates to an expired gas collecting tube which extends outside an clinical biochemical examination device. The whole transmitting system of expired gag extending to a detecting part for gas sample inside the examination device is to be similarly constructed to that of the collecting tube.

The expired gas collecting tube is assembled in a clinical examination device using expired gas as samples and feeds or transmits expired gas breathed out by subjects to the detecting part or stages prior thereto in the examination device. The expired gas collecting tube may comprise a heating type flexible tube or hard pipe in a suitable length, for example, of 0.1 to 2m and an expired gas collecting mask or mouthpiece mounted to the utmost end of the collecting tube. The collecting tube employing the flexible tube is more readily usable and general, but the hard pipe may be applicable depending on specific kinds and configuration of the examination device. The hard pipe when adapted to be shorter in length is readily usable and may include therein a rotary joint or the like in the heating type similarly to the collecting tube.

The heating type flexible tube may comprise an elastic tube provided with a heating element on the outer or inner peripheral part or inside the tube and a heat insulating material covering the outer side of the elastic tube. Materials for the elastic tube are rubber or stable resin superior in heat-resistance and not emitting gases upon heating such as Teflon resin or silicone resin. Teflon is more preferable for the purpose. The elastic tube is 1 to 5mm in inner diameter and 1 to 3mm in thickness and may become thicker when integrally incorporating therein the heating element. The heating element may be in the form of line or face as known, and the heat insulating material may use resin, fiber or rubber as known, superior in heat-resistance. For providing controllability, the flexible tube itself is preferably highly flexible as possible and also preferably thinner, for example, about 5 to 20mm in diameter. To prevent clogging from deformation of the elastic tube when the flexible tube is folded, the elastic tube is to be reinforced circumferentially. Also, in stead of use of the heating element, an elastic tube having heating efficiency obtained by molding any material similar to the above having heating efficiency by itself such as Teflon mixed with finely divided particles of metal may be covered on the outer peripheral part with a heat insulating material.

This type of expired gas collecting tube comprises a heating type flexible tube 0.5 to 2m in length provided at the utmost and with a mounting part for mounting the expired gas collecting mask or mouthpiece. The mounting part serves also as a grip of the expired gas collecting tube. Also, the expired gas collecting mask and mouthpiece may be disposable type for sanitary purpose. In this case, the mask or mouthpiece is to be freely detachable from the mounting part. The expired gas collecting tube is so heated by electrical control from the outside that the inner wall surface of the elastic tube is increased of temperature to the same as of human body or higher, for example, to 36 to 100°C, preferably to 10 to 50°C.

In case of the heating type hard pipe, a heating element is disposed circumferentially of a pipe made of metal or thermosetting resin or integrally incorporated inside the pipe, and the pipe is circumferentially covered with a heat insulating material. The heating element may be in the form or line or face as known, and the heat insulating material may use resin, fiber or rubber as known, superior in heat-resistance. Total diameter of the hard pipe is 5 to 20mm. Also, in stead of use of the heating element, an hard pipe having heating efficiency obtained by molding any material similar to the above having heating efficiency by itself such as phenol resin mixed with carbon or finely divided particles of metal may be covered on the outer peripheral part with a heat insulating material.

This type of expired gas collecting tube comprises a heating type hard pipe 10 to several dozens cm in length provided at the utmost end with a mounting part for mounting the expired gas collecting mask or mouthpiece. Also, the expired gas collecting mask and mouthpiece may be disposable type for sanitary purpose or alternatively be fixed to the utmost end of the collecting tube, for example, in a clinical examination device in type of patients' self-monitoring. Since the hard pipe type collecting tube cannot be folded, it may be provided with a rotary joint for changing directions of the collecting tube. Also similarly to the flexible tube, the expired gas collecting tube of hard pipe type is so heated by electrical control from the outside that the inner wall surface of the hard pipe is increased of temperature to the same as of human body or higher, for example, to 36 to 100°C, preferably to 40 to 50°C.

Expired gas may be fed to the examination device by patients blowing through the tube (using mainly the mouth-piece) or automatically taken in into the examination device side through suction by pump (the case using the expired gas collecting mask). Generally, the latter feature is adopted. In this case, it is sufficient for the feature that patients slowly breathe out to the expired gas collecting mask. Expired gas when contacts with the mask is possibly adsorbed of components or contaminated. Hence, such feature may be required that the mounting part (adapter) for the collecting mask or the mask itself is provided with a bypass or air vent through which expired gas possibly contacted with the collecting mask is discharged to the outside of the system.

This expired gas collecting tube as foregoing is provided for directing expired gas directly to the clinical examination device. The expired gas collecting tube according to the present invention may be applicable also to collect expired gas in an expired gas collecting means. This type of expired gas collecting means may comprise a heating type tube or hard pipe of several dozens cm at the most in length provided at one end with a mounting part for mounting an expired air collecting mask or mouthpiece and at the other end or near the end with an inserting part for the expired gas collecting means. In this case, a bag having an aperture may be provided at that other end near which the collecting means inserting part is formed, so that discharge of expired gas upon patients' breathing out is checked by watching swelling of the bag to collect the expired gas. Expired gas collected in the expired gas collecting means may be immediately upplied to the clinical examination device for analysis. The expired gas collecting means may use a large-sized injector, a gas bag, a syringe or the like. These members are also to have the construction that the inside can be heated to prevent aggregation of moisture.

Construction of the detector in the clinical examination device to which the expired gas collecting tube is assembled is classified as follows depending on the kinds of detector. (1) The detector itself has function to specifically detect aimed gas components. Controlled potential electrolytic gas detector, IMS (Ion mobility spectrometer) or ECD (Electron capture detector), (2) a combination of a detector detecting a wide range of gas components with high sensitivity as PID (Photo ionization detector) (IMS and ECD are applicable to this method) and a separation column, and (3) a combination of a color test paper (tape, strips) coloring through specific chemical reaction with aimed gas components in expired gas and a reflected light photometer, and a combination of color former (gel disc) and transmitted light photometer (The method is not the type using a single detector). Depending on the kinds of detectors, there may be various feeding methods for expired gas to a sample measuring mechanism determining an expired gas sample from taken expired gas and to the detector. The clinical examination device to analyze expired gas collected in the expired gas collecting means may have the similar construction to the above.

### EMBODIMENTS

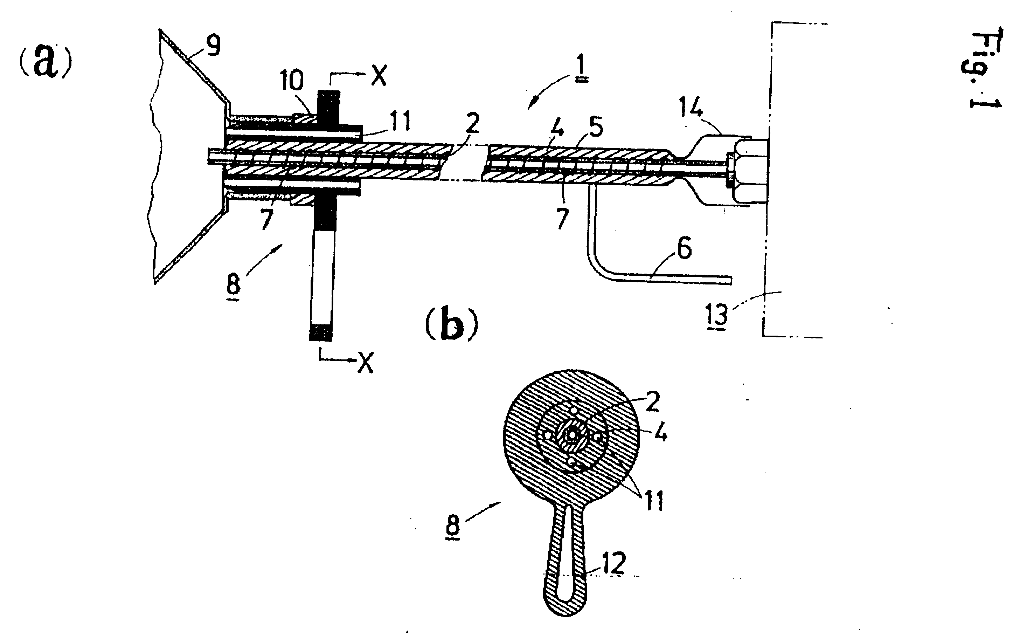

Next, the invention will be detailed with referring to the examples shown in the attached drawings. Fig. 1 shows an example of an expired gas collecting tube using a heating flexible tube type. Fig. 1(a) is a partially sectional side view and Fig. 1(b) a sectional view taken in the line X-X in Fig. 1(a). The expired gas collecting tube 1 comprises an elastic tube 2 made of Teflon, 1mm⌀ in inner diameter, 3mm⌀ in outer diameter and 1m in length, serving as a passage for expired gas. The collecting tube 1 is wound on the outer periphery with a heating line 3, covered thereon with a heat insulating material 4 of 2.5mm in thickness and further covered thereon with a plastic film cover 5 (80µm in thickness). The heating line 3 is connected with a code 6 to power source. A plurality of temperature sensors 7 are mounted on the outer surface of the elastic tube 2 to control temperatures of the inner surface of the elastic tube 2.

An adapter 8 serving also as a grip is fixed at the utmost end of the collecting tube 1 and can mount an expired gas collecting mask 9 in disposable type. 10 is a positioning stopper for the mask, 11 a bypass for discharging unnecessary expired gas to the outside of the system and 12 the grip. Rear end of the expired gas collecting tube 1 is connectable to a main body 13 of an examination device and supports an anti-folding cover 14 for the elastic tube 2.

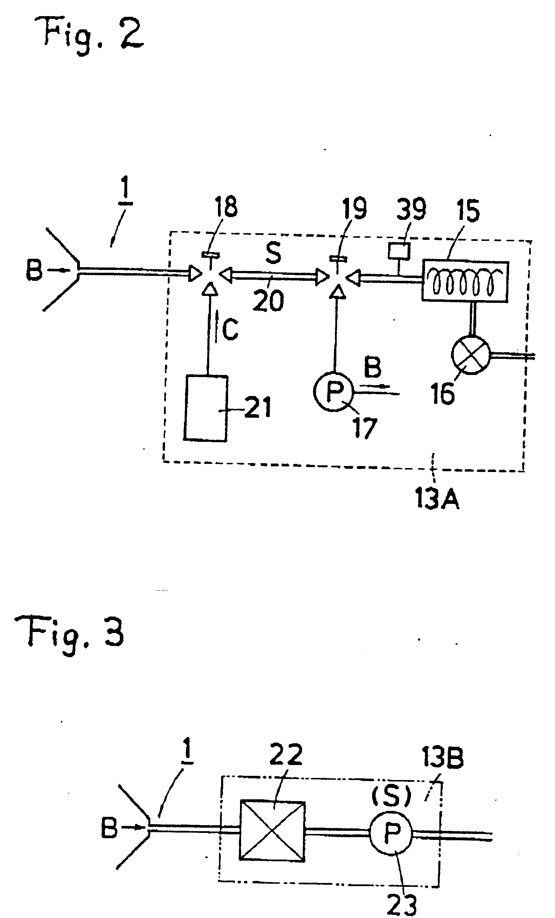

Fig. 2 shows an example of a method for connecting the expired gas collecting tube 1 to the examination device body 13. The analytical device 13A comprises a separation column 15 and a PID detector 16 and takes in expired gas B by suction pump 17 to collect an expired gas sample S in a sample measuring chamber 20 between two solenoid valves 18 and 19. The expired gas sample S is fed to the separation column 15 by a carrier gas C supplied from a cylinder 21 to be detected by the detector 16. The sample measuring chamber 20 may be kept at predetermined temperature.

The examination device body 13B shown in Fig. 3 uses IMS22 as the detecter and takes in expired gas B by a suction pump 23 while measuring the expired gas sample to carry out detection in due course. An examination device body 13C has such a different feature from the above that a color test paper 24 is sandwiched at both sides with the expired gas collecting tube 1 and a suction line 25, expired air B is measured by a suction pump 26 while being taken in, so that a reflectometer measures coloring provided by aimed gas components in the expired gas samples.

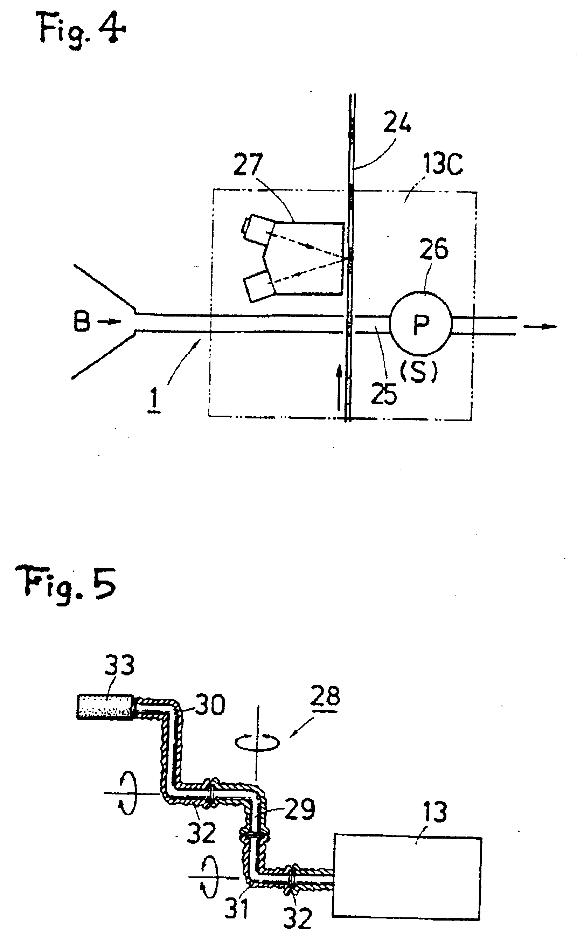

Fig. 5 shows a modified example of the expired gas collecting tube.The collecting tube 28 is a heating hard pipe type comprising a phenol resin pipe 29 serving as a passage for expired gas and provided circumferentially with a heating element 30 having faces on which element a heat insulating material 31 covers. 32 is a rotary joint. Expired gas is adapted to be blown through a mouthpiece 33 fixed at the utmost end of the collecting tube 28.

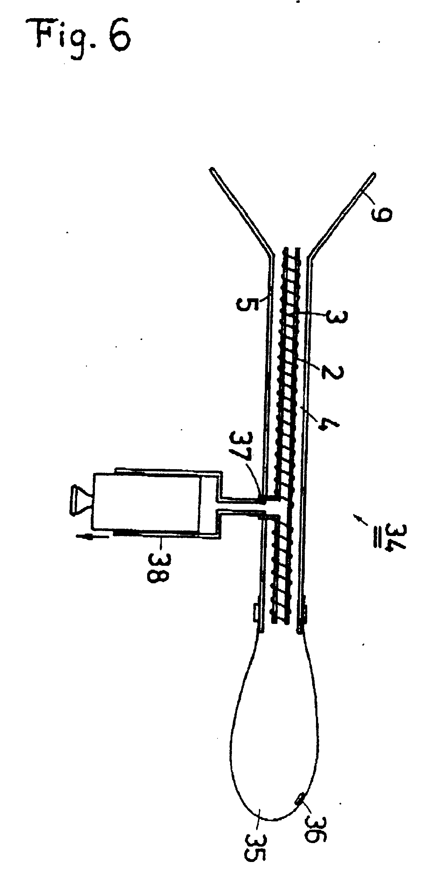

Fig. 6 shows a further modified example of the expired gas collecting tube. The collecting tube 34 comprises an elastic tube 2, 5mm⌀ in inner diameter, 8mm⌀ in outer diameter and 30cm in length, wound circumferentially with a heating line 3, covered thereon with a heat insulating material 4 or 2.5mm in thickness and further covered thereon with a plastic film cover 5 (80µm in thickness). The expired gas collecting tube 34 can mount at one end an expired gas collecting mask 9 and has at the other end a bag 35 made of thin plastic and having an aperture 36 at the end so that the bag is swollen by patients' breathing out while a part of expired gas leaks through the aperture 36 to allow the breathing-out by patients to be continued, whereby patients blowing-out expired gas can be checked by watching swelling of the bag. 37 is an inserting part for an expired gas collecting means 38.

When the expired gas collecting means 38 is a gas bag or a large-sized injector, expired gas can be fed from an expired gas collecting mask 9 (e.g., Fig. 2) to the clinical examination device. In case that the expired gas collecting means 38 is a syringe (which can quantitatively determine or measure the samples), expired gas sample may be fed through an expired gas sample injecting port 39 as shown, for example, in Fig. 2.

### EFFECTS OF THE INVENTION

As clearly seen from the above, the method according to the present invention heats the inner wall part of the expired gas collecting tube to any temperatures equal to or higher than human body temperature when expired gas is fed to a clinical examination device or an expired gas collecting means by use of the collecting tube. Also, the expired gas collecting tube according to the present invention is that for use in sampling to be carried out with a clinical examination device using expired gas as samples and comprises an elastic tube or a thermosetting resin pipe or the like circumferentially provided with a heating element and further covered thereon with a heat insulating material.

Hence, upon sampling expired gas, moisture vapor does not adhere on the inner wall of the expired gas collecting tube, so that even when a plurality of patients successively carry out measurement with the same collecting tube, there causes no contamination and an excellent reproducibility can be obtained, thereby enabling the invention to largely contribute to improvement of reliability of analysis with expired gas.

## Claims

1. An expired air sampling method wherein when expired gas is fed to a clinical examination device or an expired gas collecting means by use of an expired gas collecting tube, an inner wall part of the collecting tube is heated at temperature equal to or higher than that of human body.

2. An expired gas collecting tube in association with a clinical examination device which device causes an expired gas sample to flow through a detector and arithmetically processes outputs from the detector based on gas components to be detected for providing clinical examination data, the expired gas collecting tube comprising a heating type flexible tube which includes an elastic tube provided on outer or inner peripheral part or inside with an heating element or an elastic tube made of a material having itself heating efficiency, the elastic tube being covered circumferentially with a heat insulating material, and the heating type flexible tube being provided at the utmost end with a mounting part for mounting an expired gas collecting mask or mouth-piece.

3. An expired gas collecting tube in association with a clinical examination device which device causes an expired gas sample to flow through a detector and arithmetically processes outputs from the detector based on gas components to be detected for providing clinical examination data, the expired gas collecting tube comprising a heating type hard tube which includes a pipe provided on outer or inner peripheral part or inside with an heating element or a pipe made of a material having itself heating efficiency, the pipe being covered circumferentially with a heat insulating material, and the heating type hard tube being provided at the utmost end with a mounting part for mounting an expired gas collecting mask or mouthpiece.

4. An expired gas collecting tube for guiding expired gas to an expired gas collecting means, the expired gas collecting tube comprising a heating type flexible tube which includes an elastic tube provided on outer or inner peripheral part or inside with an heating element or an elastic tube made of a material having itself heating efficiency, the elastic tube being covered circumferentially with heat insulating material, and the heating type flexible tube being provided at one end with a mounting part for mounting an expired gas collecting mask or mouthpiece and at the other end or near the end with an inserting part for the expired gas collecting means.

5. An expired gas collecting tube for guiding expired gas to an expired gas collecting means, the expired gas collecting tube comprising a heating type hard tube which includes a pipe provided on outer or inner peripheral part or inside with an heating element or a pipe made of a material having itself heating efficiency, the pipe being covered circumferentially with a heat insulating material, and the heating type hard tube being provided at one end with a mounting part for mounting an expired gas collecting mask or mouthpiece and at the other end or near the end with an inserting part for the expired gas collecting means.
